# EUROPEAN PATENT APPLICATION

(11) **EP 4 739 043 A1**
(43) Date of publication of application: **06.05.2026**
(21) Application number: 25212125.6
(22) Date of filing: 29.10.2025
(51) Int. Cl.: H10H 20/855, H10H 29/10, H10H 29/14, H10H 29/20, H10H 29/24, H10H 29/855

(54) **LIGHT SOURCE MODULE AND ULTRAVIOLET LIGHT FLUID PROCESSING DEVICE**

(30) Priority: 01.11.2024 JP 2024193223; 30.05.2025 JP 2025091324
(71) Applicant: NICHIA CORPORATION, Tokushima 774-8601 (JP)
(72) Inventor: SANO, Hiroki, Anan-shi, Tokushima, 774-8601 (JP)
(74) Representative: Betten & Resch

(57) **Abstract**

A light source module includes a substrate, light sources positioned on an upper surface of the substrate, and an optical member including light control parts and positioned above the substrate such that the light sources are located between the substrate and the optical member.

## Description

### BACKGROUND

### 1. Field of the Invention

The present disclosure relates to a light source module and an ultraviolet light fluid processing device.

### 2. Description of the Related Art

There are known lighting devices with multiple light emitting parts. For example, Unexamined Japanese Patent Application Publication No. 2016-170912 discloses a lighting device that includes first light emitting parts composed of multiple first light emitting elements connected in series, and second light emitting parts composed of multiple second light emitting elements connected in series and emitting light in a different color from that of the first light emitting parts, and, in this lighting device, the light distribution angle of emitted light changes according to switching of the color of emitted light by turning on the first light emitting parts and the second light emitting parts selectively.

### SUMMARY

According to an embodiment of the present disclosure, a light source module includes: a substrate having a center region and an outer region located outward of the center region; light sources positioned on an upper surface of the substrate and comprising a plurality of first light emitting parts; and an optical member positioned above the substrate such that the light sources are located between the substrate and the optical member, the optical member comprising light control parts that comprise a plurality of first light control parts. Each of the plurality of first light control parts overlaps with a respective one of the plurality of first light emitting parts in a top view so as to constitute each of a plurality of first stacked bodies positioned in the outer region. In each of the plurality of first stacked bodies, the first light control part comprises: a first incident surface that is planar in shape and that faces a corresponding one of the plurality of first light emitting parts; and a first emission surface that is curved to be convex in a direction away from the first incident surface. In each of the plurality of first stacked bodies, cross sections along a first axis that passes through a center of the first emission surface in the top view and that is perpendicular to the first incident surface include a first cross section in which curvatures of both sides of the first emission surface with respect to the first axis are different from each other. In at least one of the plurality of first stacked bodies, an optical axis of a corresponding one of the plurality of first light control parts is non-parallel to the first axis.

### BRIEF DESCRIPTION OF THE DRAWINGS

FIG. 1 is a perspective view showing an example of a light source module according to a first embodiment of the present disclosure;
FIG. 2 is a partial top view (1) showing an example of the light source module of the first embodiment;
FIG. 3 is a partial top view of the light source module shown in FIG. 2 without the optical member;
FIG. 4 is a partial top view (2) showing an example of the light source module of the first embodiment;
FIG. 5 is a schematic diagram showing an enlarged view of the part labeled "A1" in FIG. 4;
FIG. 6 is a schematic diagram showing a cross section of a first stacked body S1, taken along the line VI-VI in FIG. 5;
FIG. 7 is a schematic diagram showing a cross section of the first stacked body S1, taken along the line VII-VII in FIG. 5;
FIG. 8 is a perspective view showing an example of a first light control part;
FIG. 9 is a cross-sectional view (1) showing an example of a second stacked body;
FIG. 10 is a cross-sectional view (2) showing examples of second stacked bodies;
FIG. 11 is a schematic diagram for explaining the direction in which light is emitted from the light source module of the first embodiment;
FIG. 12 is a partial top view showing an example of a light source module according to a first modified example of the first embodiment;
FIG. 13 is a schematic diagram showing an enlarged view of the part labeled "A2" in FIG. 12;
FIG. 14 is a schematic diagram showing a cross section of a third stacked body S3, taken along the line XIV-XIV in FIG. 13;
FIG. 15 is a schematic diagram showing a cross section of the third stacked body S3, taken along the line XV-XV in FIG. 13;
FIG. 16 is a schematic diagram for explaining the direction in which light is emitted from the light source module of the first modified example of the first embodiment;
FIG. 17 is a partial top view showing an example of a light source module according to a second modified example of the first embodiment;
FIG. 18 is a schematic diagram for explaining the direction in which light is emitted from the light source module of the second modified example of the first embodiment;
FIG. 19 is a partial top view showing an example of a light source module according to a third modified example of the first embodiment;
FIG. 20 is a schematic diagram for explaining the direction in which light is emitted from the light source module of the third modified example of the first embodiment;
FIG. 21 is a perspective view showing an example of an optical member for use in a light source module according to a fourth modified example of the first embodiment;
FIG. 22 is a perspective view showing an example of an ultraviolet light fluid processing device according to a second embodiment of the present disclosure;
FIG. 23 is a cross-sectional view taken along the line XXIII-XXIII in FIG. 21;
FIG. 24 is a perspective view showing an example of an ultraviolet light fluid processing device according to a first modified example of the second embodiment; and
FIG. 25 is a cross-sectional view taken along the line XXV-XXV in FIG. 24.

### DETAILED DESCRIPTION

The present disclosure aims to provide a light source module that can easily irradiate light onto a specific region.

According to at least one embodiment of the present disclosure, a light source module can easily irradiate light onto a specific region.

Hereinafter, certain embodiments of the present disclosure will be described with reference to the accompanying drawings. In the following description, terms that indicate specific directions or positions (e.g., "upper," "lower," etc.) are used where appropriate. However, these terms are used only for the purpose of facilitating understanding of the present disclosure with reference to the accompanying drawings, and the meaning of these terms does not limit the technical scope of the present invention. Also, parts that appear in multiple drawings with the same reference numerals indicate the same or equivalent parts or members.

Embodiments and examples shown hereinafter are examples of light source modules and related components for giving a concrete form to the technical idea of the present disclosure and is not intended to limit the technical scope of the present invention to description herein. Also, unless otherwise specified, the dimensions, materials, shapes, relative positions, etc. of the components described below are intended to be illustrative and are not intended to limit the technical scope of the present disclosure. The details described in relationship to one embodiment are applicable to other embodiments, modified examples, examples, etc. The size, positional relationship, etc. of the members shown in the drawings may be exaggerated for ease of explanation. Furthermore, in order to avoid the drawings from becoming excessively complicated, schematic diagrams in which certain elements are omitted may be used.

### <First Embodiment>

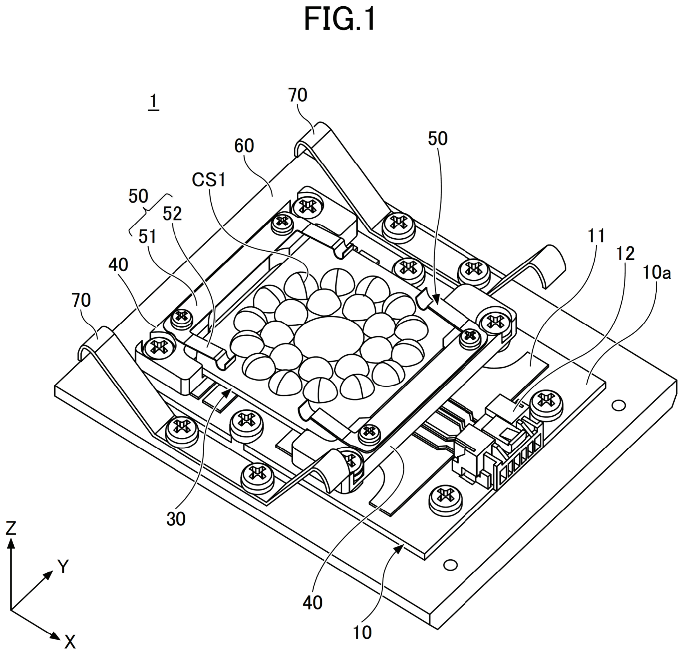

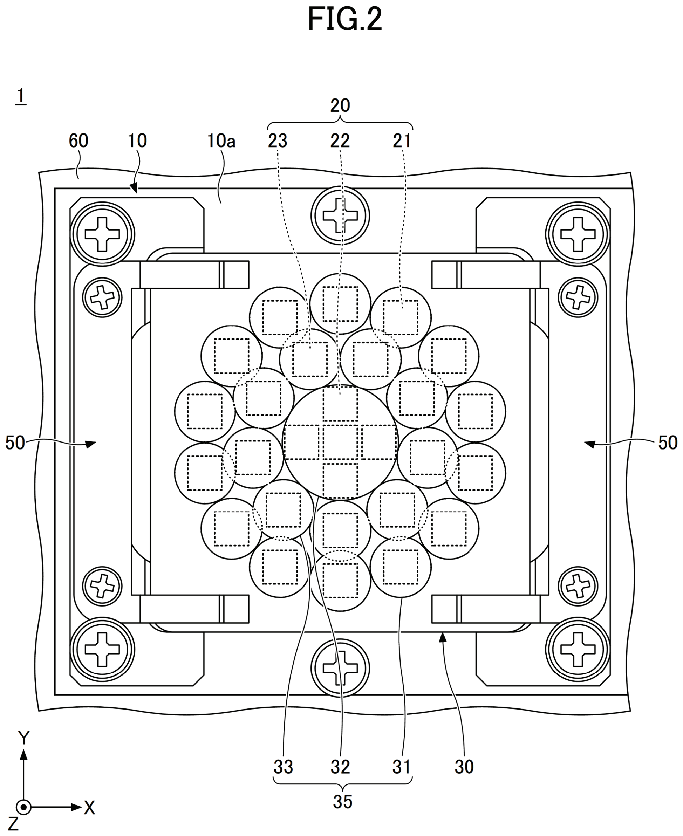

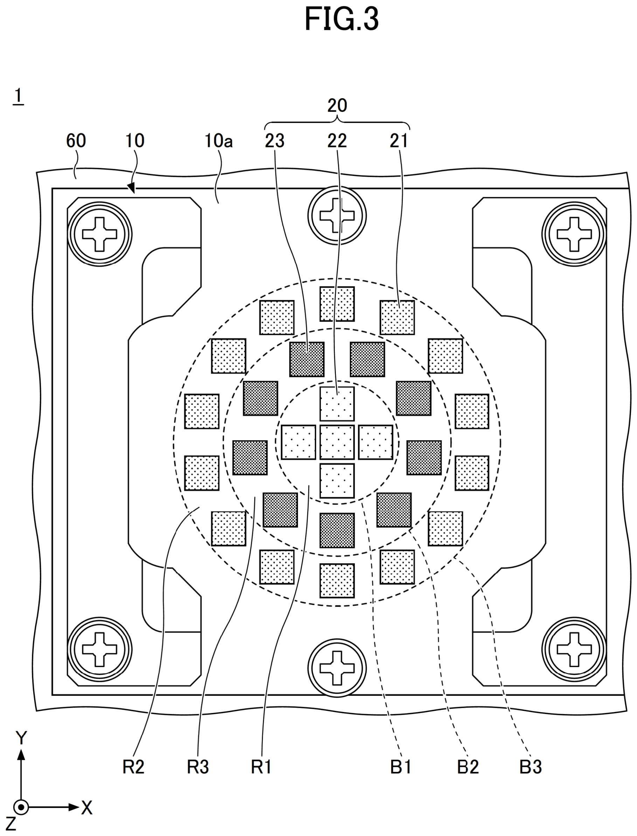

FIG. 1 is a perspective view showing an example of a light source module according to a first embodiment of the present disclosure. FIG. 2 is a partial top view (1) showing an example of the light source module of the first embodiment. FIG. 3 is a partial top view in which the optical member is removed from the light source module shown in FIG. 2.

In these drawings, an X-axis, a Y-axis, and a Z-axis that are mutually perpendicular are shown for reference. Directions that are parallel to the X-axis will be hereinafter referred to as "first direction(s) X," directions that are parallel to the Y-axis will be hereinafter referred to as "second direction(s) Y," and directions that are parallel to the Z-axis will be hereinafter referred to as "third direction(s) Z." For example, in the first directions X, the direction in which the arrow points is the "+X direction," and the direction opposite to the +X direction is the "-X direction." In the second directions Y, the direction in which the arrow points is the "+Y direction," and the direction opposite to the +Y direction is the "-Y direction." In the third directions Z, the direction in which the arrow points is the "+Z direction," and the direction opposite to the +Z direction is the "-Z direction." However, these directions do not limit the orientation of the light source module upon use, and the light source module may be positioned and oriented in any appropriate direction.

As shown in FIG. 1 to FIG. 3, the light source module 1 includes a substrate 10, light sources 20, an optical member 30, pedestal parts 40, fixing members 50, a holding member 60, and a spring member 70. The light source module 1 does not necessarily include the pedestal parts 40, the fixing members 50, the holding member 60, and the spring member 70. Note that, referring to FIG. 1, CS1 is a schematic representation of a particular cross section that is parallel to the third direction Z. The line of CS1 is a virtual line, and no such line actually exists. CS1 will be described in detail later.

The substrate 10 is, for example, rectangular in top view. In the illustrated examples, the upper surface 10a and the lower surface of the substrate 10 are flat surfaces, and the long sides of the upper surface 10a are parallel to the first direction X, and the short sides are parallel to the second direction Y. Also, the normal to the upper surface 10a is parallel to the third direction Z. However, the substrate 10 is not limited to be shaped rectangular in top view. The substrate 10 has a predetermined pattern of a conductive member 11. A connector 12 that is electrically connected to the conductive member 11 may or may not be arranged on the upper surface 10a of the substrate 10. Unless otherwise specified, "top view" used herein means a view of the light source module 1 in the direction normal to the upper surface 10a of the substrate 10.

The light sources 20 are positioned on the upper surface 10a of the substrate 10. The optical member 30 is positioned above the substrate 10, with the light sources 20 between the optical member 30 and the substrate 10 in the Z direction. The optical member 30 may or may not be in contact with the light sources 20. The light sources 20 and the optical member 30 will be described in detail later.

In the illustrated examples, a pair of pedestal parts 40 are fixed onto the substrate 10, and the optical member 30 is held by the pedestal parts 40. To be more specific, a pair of fixing members 50 are provided on the respective upper surfaces of the pedestal parts 40 such that, in a top view, the fixing members 50 face each other with the optical member 30 disposed therebetween.

The fixing members 50 are, for example, metal plates. Each fixing members 50 includes, for example, a fixing part 51 and spring parts 52. Each fixing part 51 extends in the short-side direction (Y direction) of the upper surface 10a of the substrate 10 and is fixed to the pedestal part 40 below it. The spring parts 52 extend from both ends of the fixing part 51, onto the upper surface of the optical member 30. Each fixing part 51 can be fixed to the upper surface of the pedestal part 40 by, for example, screwing. The fixing members 50 press the optical member 30 against the substrate 10, so that the optical member 30 is held by the pedestal parts 40.

The substrate 10, on which the light sources 20 and the optical member 30 are arranged, can be fixed, for example, on a plate-shaped holding member 60. The substrate 10 can be fixed on the upper surface of the holding member 60, for example, by screwing, by an adhesive, etc.

Two spring members 70, extending in the long-side direction (X direction) of the upper surface 10a of the substrate 10, are positioned on the holding member 60. The spring members 70 can be positioned such that they face each other with the substrate 10 therebetween in a top view. The spring members 70 are, for example, flat springs made of metal. The spring members 70 can be fixed to the upper surface of the holding member 60 by, for example, screwing. The number of spring members 70 is not limited to two and may be any number greater than or equal to one. The spring members 70 are not limited to be in the shape shown in FIG. 1 and may be in any appropriate shape. The spring members 70 can be used when fixing the light source module 1 in a predetermined space.

The light source module 1 may have a jack mechanism instead of the spring members 70. This jack mechanism may be, for example, configured such that, for example, rotation of a spring member causes a support member to move upward and downward relative to the upper surface of the holding member 60. For example, the light source module 1 can be fixed in a predetermined space by inserting the light source module 1 in a predetermined space in a state in which the support member has been lowered and then rotating the spring member to raise the support member.

As shown in FIG. 3, the substrate 10 at least includes a center region R1 and an outer region R2 located outward of the center region R1, which are regions where the light sources 20 are positioned. The center region R1 and the outer region R2 are regions on the upper surface 10a of the substrate 10.

In the example illustrated in FIG. 3, the substrate 10 further includes a middle region R3 located between the center region R1 and the outer region R2. The center region R1 is located closer to the center than is a boundary B1. The outer region R2 is located between a boundary B2 and a boundary B3. The middle region R3 is located between the boundary B1 and the boundary B2. The boundary B1 is located inward of the boundary B2. The boundary B2 is located inward of the boundary B3 and outward of the boundary B1. In FIG. 3, the boundary B1, the boundary B2, and the boundary B3 are shown with dashed lines, and these lines are virtual lines.

The light sources 20 include a plurality of first light emitting parts 21. In the examples shown in FIG. 1 to FIG. 3, the light sources 20 further include one or more second light emitting parts 22 and a plurality of third light emitting parts 23. Referring to FIG. 3, for ease of understanding, all the first light emitting parts 21 are shown in a dot pattern of a unique density, all the second light emitting parts 22 are shown in a dot pattern of another unique density, and all the third light emitting parts 23 are shown in a dot pattern of yet another unique density. The patterns of the first light emitting parts 21, the second light emitting parts 22, and the third light emitting parts 23 vary in the density of dots.

In a top view, the first light emitting parts 21 are positioned in the outer region R2. One or more second light emitting parts 22 are positioned in the center region R1. In a top view, the third light emitting parts 23 are positioned in the middle region R3. The first light emitting parts 21, the second light emitting parts 22, and the third light emitting parts 23 are connected in series with each other via the conductive member 11. The first light emitting parts 21 are not positioned in the center region R1. The second light emitting parts 22 are not positioned in the outer region R2. The first light emitting parts 21 and the second light emitting parts 22 are not positioned in the middle region R3. The third light emitting parts 23 are not positioned in the center region R1 or the outer region R2.

For example, the number of first light emitting parts 21 positioned in the outer region R2 is greater than the number of second light emitting parts 22 positioned in the center region R1. The number of third light emitting parts 23 positioned in the middle region R3 is greater than the number of second light emitting parts 22 positioned in the center region R1 and less than the number of first light emitting parts 21 positioned in the outer region R2.

The first light emitting parts 21, the second light emitting parts 22, and the third light emitting parts 23 are positioned in different regions in the example illustrated in FIG. 3 but have the same configuration. The "same configuration" used herein means exhibiting at least the same wavelength range of emitted light, luminous intensity, and light distribution angle. The first light emitting parts 21, the second light emitting parts 22, and the third light emitting parts 23 emit, for example, ultraviolet light. In this case, the light source module 1 can emit ultraviolet light from the optical member 30. The first light emitting parts 21, the second light emitting parts 22, and the third light emitting parts 23 may emit light of a wavelength range other than that of ultraviolet light.

When the first light emitting parts 21, the second light emitting parts 22, and the third light emitting parts 23 emit ultraviolet light, the peak wavelength of the ultraviolet light is between, for example, 10 nm and 405 nm, inclusive. The difference in the peak wavelength of emitted light is preferably within 5 nm among the first light emitting part 21, the second light emitting part 22, and the third light emitting part 23. The first light emitting parts 21, the second light emitting parts 22, and the third light emitting parts 23 include light emitting elements. The light emitting elements may be, for example, light emitting diodes (LEDs) or laser diodes (LDs). As each of the first light emitting part 21, the second light emitting part 22, and the third light emitting part 23, for example, a light emitting device in which a light emitting element is arranged in a package can be used. The package may be made of a ceramic material and/or a resin material.

The first light emitting part 21, the second light emitting part 22, and the third light emitting part 23 may emit lights of different wavelengths. For example, the first light emitting parts 21, the second light emitting parts 22, and the third light emitting parts 23 may include two or more light emitting parts that emit light having a peak wavelength between 260 nm and 280 nm, inclusive, light emitting parts that emit light having a peak wavelength greater than 280 nm and less than 315 nm, and light emitting parts that emit light having a peak wavelength between 315 nm and 405 nm, inclusive. In one example in which these three types of light emitting parts are used, for example, the first light emitting parts 21 emits light having a peak wavelength between 260 nm and 280 nm, inclusive, the second light emitting parts 22 emits light having a peak wavelength between 315 nm and 405 nm, inclusive, and the third light emitting parts 23 emits light having a peak wavelength greater than 280 nm and less than 315 nm. Also, the peak wavelength of emitted light may be interchanged between the first light emitting parts 21 and the second light emitting parts 22, between the first light emitting parts 21 and the third light emitting parts 23, or between the second light emitting parts 22 and the third light emitting parts 23.

In the examples shown in FIG. 1 to FIG. 3, fourteen first light emitting parts 21 are positioned in the outer region R2. Five second light emitting parts 22 are positioned in the center region R1. Nine third light emitting parts 23 are positioned in the middle region R3. The number of light emitting parts positioned in each region is not limited to the examples shown in FIG. 1 to FIG. 3.

The optical member 30 has a square outer shape in a top view. The optical member 30 may have a rectangular, circular, or elliptical outer shape in top view. The terms "square" and "rectangle" used herein may encompass ones with chamfered or rounded corners in addition to ones with right-angled corners.

The optical member 30 includes light control parts 35. The optical member 30 has an upper surface and a lower surface, and the light control parts 35 are positioned on the upper surface of the optical member 30. The light control parts 35 are protruding parts that protrude toward a side opposite to the light sources 20. In a top view, the thickness of each protruding part is greatest at its center part and is gradually reduced away from the center part. The thickness of each protruding part refers to the distance from the upper surface of the optical member 30 to the surface of the protruding part in the direction normal to the upper surface of the optical member 30.

The light control parts 35 (protruding parts) can function as convex lenses. In the examples shown in FIG. 1 to FIG. 3, each of the protruding parts has a circular shape in a top view. The optical member 30 can be made of, for example, glass or resin. The glass may be, for example, quartz glass, borosilicate glass, etc. The resin may be, for example, silicone-based resin.

The light control parts 35 includes a plurality of first light control parts 31. In the example shown in FIG. 1 to FIG. 3, the light control parts 35 further include one or more second light control parts 32 and a plurality of third light control parts 33. The first light control parts 31 are positioned in an outer region in the optical member 30 in a top view. The second light control parts 32 are positioned in a center region in the optical member 30 in a top view. The third light control parts 33 are positioned in a middle region, which is located between the center region and the outer region in the optical member 30 in a top view.

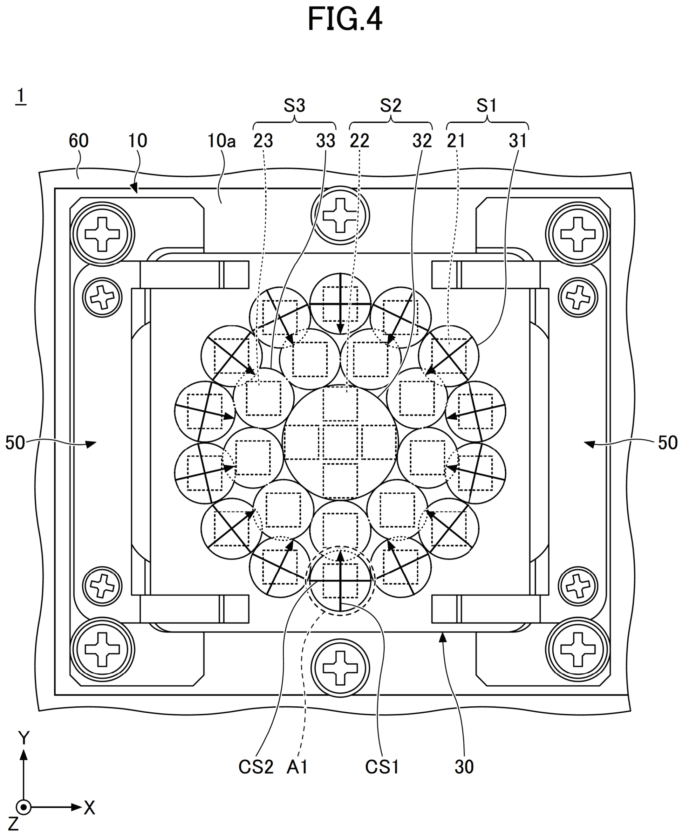

For example, assuming that a single light control part of the light control parts 35 has a circular outer edge in a top view, circles that correspond to such outer edges of adjacent light control parts may or may not have portions overlapping with each other when seen from above. Although the circles corresponding to outer edges of the first light control parts 31 that are adjacent to each other in a top view do not have overlapping portions in the example of FIG. 2, these circles may have overlapping portions. Likewise, although the circles corresponding to outer edges of the third light control parts 33 that are adjacent to each other in a top view have overlapping portions in FIG. 2, these circles do not have to overlap with each other. Although circles corresponding to outer edges of some first light control parts 31 overlap with circles corresponding to outer edges of third light control parts 33 adjacent to them in a top view in FIG. 2, these circles do not have to overlap. Although the circle corresponding to an outer edge of the second light control part 32 and the circles corresponding to outer shapes of third light control parts 33 adjacent to it have no overlapping part in top view in FIG. 2, the circle of the second light control part and the circles of the third light control parts 33 may overlap with each other. In FIG. 2, it is assumed that an outer edge of a single light control part has a circular shape in a top view, and circular arcs that correspond to overlapping portions of such outer edges of light control parts adjacent to each other are illustrated using dashed lines. The same applies to FIG. 4, FIG. 5, FIG. 6, FIG. 12, FIG. 13, FIG. 14, FIG. 17, and FIG. 19, which will be described later.

Light emitted from one of the first light emitting parts 21 is incident on a first light control part 31 that overlaps with it in a top view. A light distribution angle of the incident light is narrowed by the protruding part of the first light control part 31, and the light exits the first light control part 31 on a side (the upper surface) opposite to the substrate 10. Light emitted from one or more of the second light emitting parts 22 is incident on the second light control part 32 that overlaps with them in a top view. A light distribution angle of the incident light is narrowed by the protruding part of the second light control part 32, and the light exits the second light control part 32 on a side opposite to the substrate 10. Light emitted from one of the third light emitting parts 23 that coincides with the third light control part 33 in a top view is incident on each of the third light control parts 33. The light distribution angle of the incident light is narrowed by the protruding part of the third light control part 33, and the light exits the third light control part 33 on a side opposite to the substrate 10.

The thickness of the protruding part may be the same or different among the first light control parts 31, the second light control parts 32, and the third light control parts 33. The area of the protruding part in a top view may be the same or different among the first light control parts 31, the second light control parts 32, and the third light control parts 33. The curvature of the protruding part may be the same or different among the first light control parts 31, the second light control parts 32, and the third light control parts 33. The light distribution angle can be adjusted by changing the thicknesses of the protruding part, the area of the protruding part in a top view, and/or the curvature of the protruding part.

In the illustrated examples, each first light control part 31 of the optical member 30 coincides with a unique first light emitting part 21 in a top view, and the second light control part 32 of the optical member 30 overlaps with five second light emitting parts 22 in top view. With this structure, a light source module with an optical member 30 can be realized, in which the first light emitting parts 21 positioned in the outer region R2 are optically controlled individually by respective first light control parts 31, and in which the five second light emitting parts 22 positioned in the center region R1 are optically controlled collectively by a single second light control part 32.

Also, the light source module 1 may include a plurality of third light emitting parts 23 and multiple third light control parts 33 as needed, and each third light control part 33 may be positioned to overlap with a respective one of third light emitting parts 23 in top view. With this structure, a light source module having an optical member 30, in which the light sources 20 are formed with first light emitting parts 21, second light emitting parts 22, and third light emitting parts 23 arranged as described above, can be provided.

FIG. 4 is a partial top view (2) showing an example of the light source module according to the first embodiment. As shown in FIG. 4, each first light control part 31 overlaps with a respective one of the first light emitting parts 21 in a top view so as to constitute a first stacked body S1 together. The first stacked bodies S1 formed thus are positioned in the outer region R2 as shown in FIG. 3. Likewise, one or more second light control parts 32 overlap with one or more second light emitting parts 22 in a top view to constitute second stacked bodies S2 such that the number of second light control parts 32 and the number of second stacked bodies S2 are the same. In the illustrated examples, one second light control part 32 overlaps with five second light emitting parts 22 in a top view, constituting one second stacked body S2 together. The second stacked body S2 is positioned in the center region R1 in FIG. 3. Also, in a top view, each third light control part 33 overlaps with a respective one of the third light emitting parts 23 so as to constitute a third stacked body S3 together. The third stacked bodies S3 formed thus are positioned in the middle region R3 shown in FIG. 3.

In FIG. 4, CS1 and CS2 indicate the positions of specific cross sections that are parallel to the third direction Z. The lines of CS1 and CS2 are virtual lines. The same is true for the other figures. CS1 and CS2 will be described in detail later.

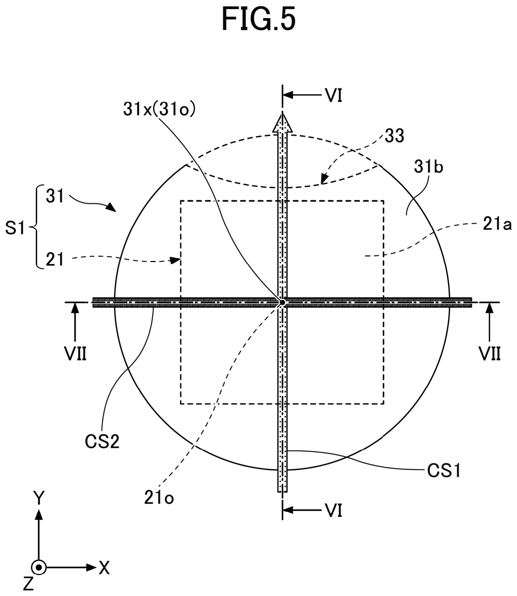

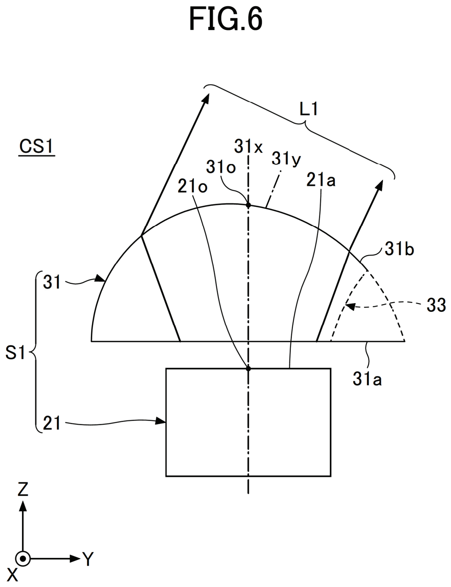

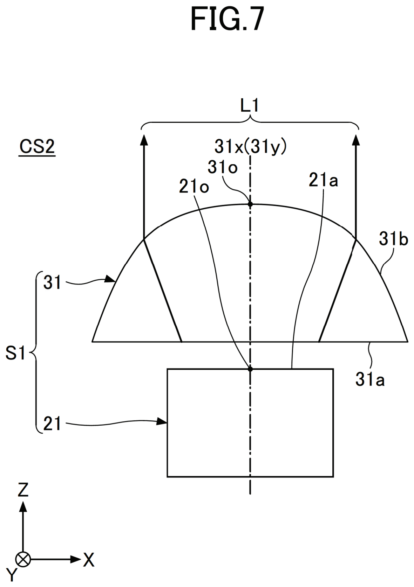

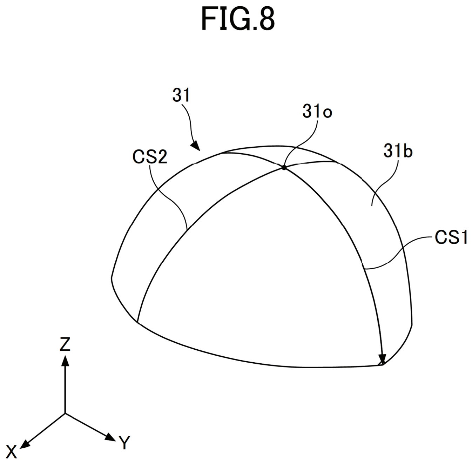

FIG. 5 is a schematic diagram showing an enlarged view of the part labeled "A1" in FIG. 4. FIG. 6 is a schematic diagram showing a cross section of a first stacked body S1, taken along the line VI-VI in FIG. 5. The line VI-VI is parallel to the second direction Y. The cross section taken along the line VI-VI thus is the first cross section CS1. That is, FIG. 6 shows the first cross section CS1. FIG. 7 is a schematic diagram showing a cross section of the first stacked body S1, taken along the line VII-VII in FIG. 5. The line VII-VII is parallel to the first direction X. The cross section taken along the line VII-VII is the second cross section CS2. That is, FIG. 7 shows the second cross section CS2. FIG. 8 is a perspective view showing an example of a first light control part. The first cross section CS1 and the second cross section CS2 are perpendicular to each other.

In FIG. 5 to FIG. 8, "21a" represents a light emitting surface of a first light emitting part 21, and "21o" represents the center of the light emitting surface 21a of the first light emitting part 21 in a top view. Also, "31a" represents a first incident surface of a first light control part 31, which can be planar, for example, "31b" represents a first emission surface of the first light control part 31, and "31o" represents the center of the first emission surface 31b in a top view. Also, "31x" represents a first axis, which passes through the center 31o of the first emission surface 31b and is perpendicular to the first incident surface 31a. "31y" represents the optical axis of the first light control part 31. The optical axis 31y serves as an optical path of light emitted from the center 21o of the light emitting surface 21a of the first light emitting part 21. FIG. 6 and FIG. 7 are cross sections each passing through the center 21o of the light emitting surface 21a of the first light emitting part 21 and taken along the first axis 31x. The center of a light emitting surface in top view herein means the geometric center of the light emitting surface in top view. Similarly, the center of an emission surface in top view herein means the geometric center of the emission surface in top view.

As shown in FIG. 5 to FIG. 8, in each first stacked body S1, the first light control part 31 has a first incident surface 31a, which is planar in shape and faces the first light emitting part 21, and a first emission surface 31b, which is curved to be convex in a direction away from the first incident surface 31a.. In each first stacked body S1, the cross sections taken along the first axis 31x include a first cross section CS1, in which curvatures of both sides of the first emission surface 31b with respect to the first axis 31x are different from each other. The term "different curvatures" in the present specification refers to a case in which the larger one of two varying curvatures is greater than 102% of the smaller one of the two varying curvatures.
The term "the same curvature" in the present specification refers to a case in which the larger one is 102% or less of the smaller one.

In the example of the first cross section CS1 shown in FIG. 6, the part of the first emission surface 31b located closer to the center region R1 shown in FIG. 3 relative to the first axis 31x (that is, the +Y half of the first emission surface 31b in FIG. 6) has a smaller curvature than that of the part of the first emission surface 31b located farther from the center region R1 shown in FIG. 3 relative to the first axis 31x (that is, the -Y half of the first emission surface 31b in FIG. 6).

When each of at least one first stacked body S1 thus includes a first cross section CS1 in which both sides of the first emission surface 31b with respect to the first axis 31x have curvatures different from each other, the optical axis 31y of the first light control part 31 can be non-parallel to the first axis 31x. As a result of this, the light source module 1 can easily irradiate light onto a specific region. As used herein, "parallel" refers to, for example, a case in which the shift between two axes in question is less than 2 degrees. Conversely, "non-parallel" refers to, for example, a case in which the shift between two axes in question is 2 degrees or greater.

A first cross section CS1, in which both sides of the first emission surface 31b with respect to the first axis 31x have curvatures different from each other, may or may not be included in all of the first stacked bodies S1. When all of the first stacked bodies S1 include a first cross section CS1 in which both sides of the first emission surface 31b with respect to the first axis 31x have curvatures different from each other, the optical axis 31y of the first light control part 31 can be non-parallel to the first axis 31x in all of the first stacked bodies S1. Further, by adjusting the orientation of the first cross section CS1, in which curvatures of both sides of the first emission surface 31b with respect to the first axis 31x are different from each other, in a top view in each of the first stacked bodies S1, light can be easily irradiated from all of the first stacked bodies S1 toward a predetermined region.

The center 21o of the light emitting surface 21a of the first light emitting part 21 may or may not be on the first cross section CS1 in top view in at least one of the first stacked bodies S1. The center 21o of the light emitting surface 21a of the first light emitting part 21 may or may not be on the first cross section CS1 in a top view in each of all of the first stacked bodies S1.

The first axis 31x may or may not pass through the center 21o of the light emitting surface 21a of the first light emitting part 21 in at least one of the first stacked bodies S1. The first axis 31x may or may not pass through the center 21o of the light emitting surface 21a of the first light emitting part 21 in each of all of the first stacked bodies S1.

In some or all of the first stacked bodies S1, the first axis 31x may or may not pass through the center 21o of the light emitting surface 21a of the first light emitting part 21. In other words, the center 31o and the center 21o may or may not coincide with each other in a top view. For example, the positional relationship between a first light emitting part 21 and its corresponding first light control part 31 can be adjusted within the range in which the center 21o of the light emitting surface 21a and the first emission surface 31b of the first light control part 31 overlap with each other in top view. Adjusting the positional relationship between the center 31o and the center 21o in a top view in addition to having curvatures different between both sides of the first emission surface 31b with respect to the first axis 31x in the first cross section CS1 allows for changing the extent that the optical axis 31y of the first light control part 31 is non-parallel to the first axis 31x.

In the second cross section CS2 shown in FIG. 7, both sides of the first emission surface 31b with respect to the first axis 31x have the same curvature. As shown in FIG. 2 and other figures, when circles corresponding to outer edges of adjacent light control parts have overlapping parts when viewed from above, such a shape is encompassed in the shape in which both sides of the first emission surface 31b with respect to the first axis 31x have the same curvature. Thus, in each first stacked body S1, the cross sections taken along the first axis 31x may include a second cross section CS2, which has the same curvature at both sides of the first emission surface 31b with respect to the first axis 31x and is perpendicular to the first cross section CS1. Both sides of the first emission surface 31b with respect to an axis that passes through the first cross section CS1 and that is perpendicular to the first incident surface 31a may have the same curvature in all of the cross sections that are parallel to the second cross section CS2. Also, in all of the cross sections that are parallel to the first cross section CS1, the curvature of the first emission surface 31b may different between both sides of the first emission surface 31b with respect to an axis that passes through the second cross section CS2 and that is perpendicular to the first incident surface 31a.

In FIG. 4, FIG. 5, and FIG. 8, the arrow of the first cross section CS1 points to the side of the first emission surface 31b having the smaller curvature. In the example of FIG. 4, each of the first stacked bodies S1 include a first cross section CS1 and a second cross section CS2. Then, in the first cross section CS1 in all of the first stacked bodies S1, the part of the first emission surface 31b located closer to the center region R1 shown in FIG. 3 relative to the first axis 31x has a smaller curvature than that of the part of the first emission surface 31b located farther from the center region R1 shown in FIG. 3 relative to the first axis 31x. With this structure, in the light source module 1, light from all of the first stacked bodies S1 can be easily irradiated toward the center of the center region R1.

Referring to FIG. 4, the extending line of the first cross section CS1 at least one of the first stacked bodies S1 passes through the center region R1 shown in FIG. 3 in a top view. The extending line of the first cross section CS1 may or may not pass through the center region R1 shown in FIG. 3 in a top view in all of the first stacked bodies S1. In a top view, the respective extending lines from the first cross sections CS1 of all first stacked bodies S1 may or may not intersect at one point. When the respective extending lines from the first cross sections CS1 of all first stacked bodies S1 intersect at one point, the lights emitted from individual first stacked bodies S1 can be concentrated in a narrower area.

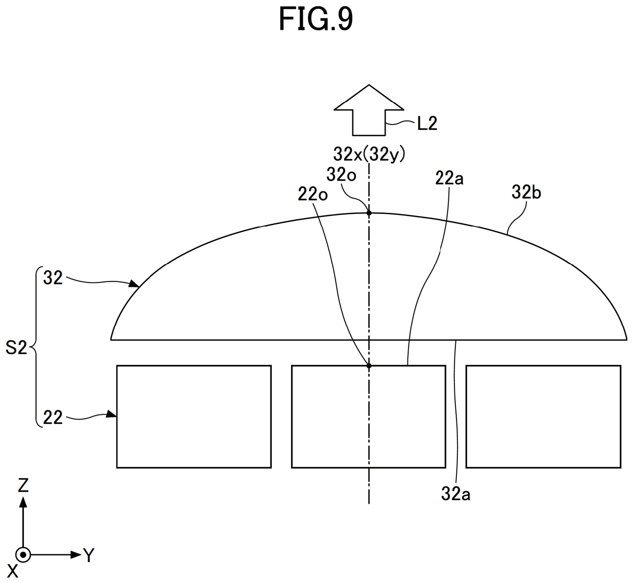

FIG. 9 is a cross-sectional view (1) showing an example of a second stacked body. FIG. 9 shows a cross section that passes through the center of a second light control part 32 in top view in FIG. 2, and that is parallel to the Y-Z plane. Referring to FIG. 9, "22a" represents a light emitting surface of a second light emitting part 22, and "22o" represents the center of the light emitting surface 22a of the second light emitting part 22 viewed from above. Also, "32a" represents a second incident surface of the second light control part 32, "32b" represents a second emission surface of the second light control part 32, and "32o" represents the center of the second emission surface 32b when viewed from above. Also, "32x" represents a second axis, which passes through the center 32o of the second emission surface 32b and is perpendicular to the second incident surface 32a. "32y" represents the optical axis of the second light control part 32. The optical axis 32y serves as a path of the light emitted from the center 22o of the light emitting surface 22a of the second light emitting part 22.

As shown in FIG. 9, in each second stacked body S2, the second light control part 32 has a second incident surface 32a, which is planar in shape and faces the second light emitting part 22, and a second emission surface 32b, which is curved to be convex in a direction away from the second incident surface 32a. In this case, in all of the cross sections taken along the second axis 32x that passes through the center 32o of the second emission surface 32b in a top view and that is perpendicular to the second incident surface 32a, the curvature of the second emission surface 32b can be the same on both sides of the second emission surface 32b with respect to the second axis 32x. In other words, the second light control part 32 in a state of a single second light control part 32 not overlapping an adjacent light control part is preferably rotationally symmetrical with respect to the second axis 32x. In the second stacked body S2 shown in FIG. 9, the optical axis of the second light control part 32 is parallel to the third direction Z.

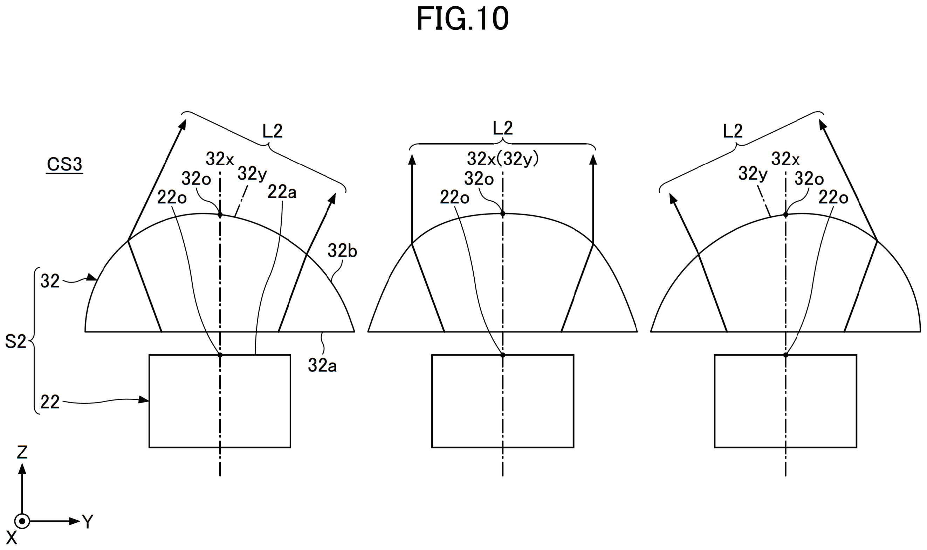

FIG. 10 is a cross-sectional view (2) showing examples of second stacked bodies. Referring to FIG. 10, when a light source module includes a plurality of second stacked bodies S2, the optical axis of the second light control part 32 may be parallel to the second axis 32x in each of all of the second stacked bodies S2, but it is preferable that the optical axis of the second light control part 32 is non-parallel to the second axis 32x in at least one of the second stacked bodies S2. In both cases of FIG. 9 and FIG. 10, light can be easily emitted from the second stacked bodies S2 toward the center of the center region R1.

In the example shown in FIG. 10, the second stacked bodies S2 other than the one at the center include a third cross section CS3, in which both sides of the second emission surface 32b with respect to the second axis 32x have different curvatures. In the second stacked bodies S2 other than the one at the center, the part of the second emission surface 32b located closer to the center region R1 shown in FIG. 3 relative to the second axis 32x has a smaller curvature than that of the part of the second emission surface 32b located farther from the center region R1 shown in FIG. 3 relative to the second axis 32x. With this structure, in the second stacked bodies S2 other than the one at the center, the optical axis of the second light control part 32 can be non-parallel to the second axis 32x. In both cases of FIG. 9 and FIG. 10, the second stacked bodies S2 can emit light towards the center of the center region R1 with ease.

In the light source module 1, all the third stacked bodies S3 emit light in directions parallel to the third direction Z.

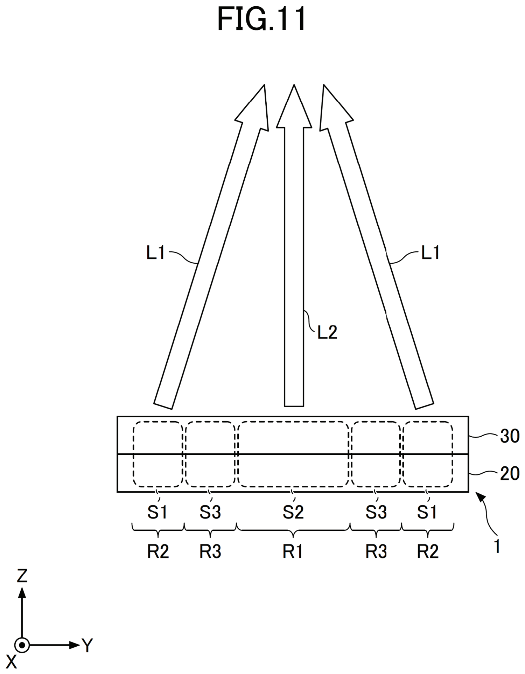

Thus, in the light source module 1, light can be easily emitted from the first stacked bodies S1 toward the center of the center region R1, as schematically illustrated in FIG. 11. Also, the second stacked bodies S2 can emit light towards the center of the center region R1. This is effective when, for example, an object to be irradiated with light exists in the direction through the center of the center region R1.

In each first stacked body S1, the part of the first emission surface 31b located nearer to the center region R1 shown in FIG. 3 relative to the first axis 31x can have a larger curvature than that of the part of the first emission surface 31b located farther from the center region R1 shown in FIG. 3 relative to the first axis 31x. In this case, the first stacked bodies S1 can emit light in directions away from the center region R1. This is effective when, for example, there is an object to be irradiated with light in a direction diverging from the center region R1.

### <First Modified Example of First Embodiment>

In a first modified example of the first embodiment, a light source module will be described below, in which the shape of the protruding part of the third light control parts 33 in the third stacked bodies S3 is different from that of the first embodiment. In the following first modified example of the first embodiment, the first stacked bodies S1 and the second stacked bodies S2 are the same as those of the first embodiment.

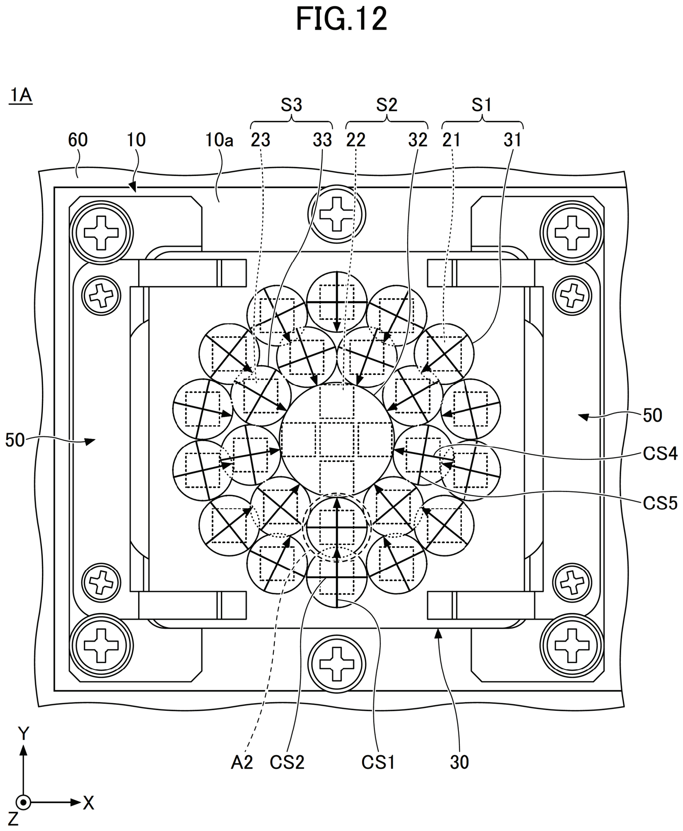

FIG. 12 is a partial top view showing an example of the light source module according to the first modified example of the first embodiment. In FIG. 12, CS4 and CS5 represent, schematically, the positions of particular cross sections that are parallel to the third direction Z. The lines of CS4 and CS5 are virtual lines. The same applies to other figures. CS4 and CS5 will be described in detail later.

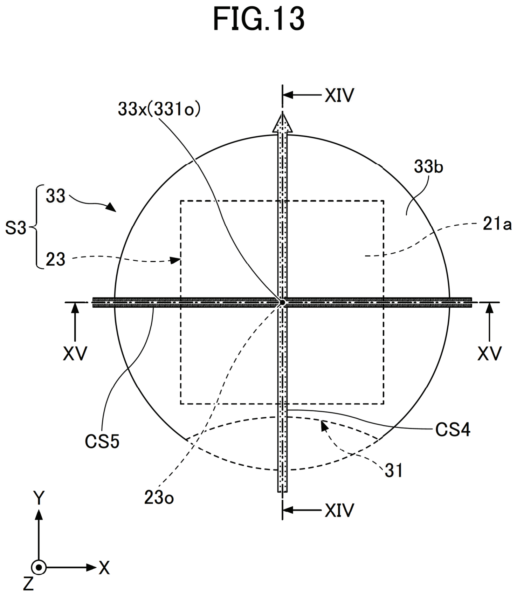

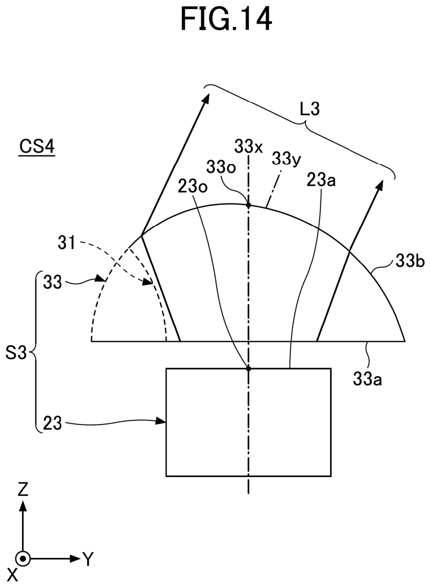

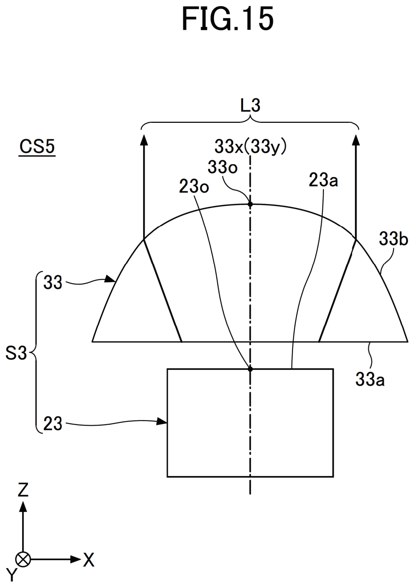

FIG. 13 is a schematic diagram showing an enlarged view of the part labeled "A2" in FIG. 12. FIG. 14 is a schematic diagram showing a cross section of a third stacked body S3, taken along the line XIV-XIV in FIG. 13. The line XIV-XIV is parallel to the second direction Y. The cross section taken along the line XIV-XIV is the fourth cross section CS4. That is, FIG. 14 shows the fourth cross section CS4. FIG. 15 is a schematic diagram showing a cross section of the third stacked body S3, taken along the line XV-XV in FIG. 13. The line XV-XV is parallel to the first direction X. The cross section taken along the line XV-XV is the fifth cross section CS5. That is, FIG. 15 shows the fifth cross section CS5. The fourth cross section CS4 and the fifth cross section CS5 are perpendicular to each other.

In FIG. 12 to FIG. 15, "23a" represents a light emitting surface of a third light emitting part 23, and "23o" represents the center of the light emitting surface 23a of the third light emitting part 23 in top view. Also, "33a" represents a third incident surface of a third light control part 33, "33b" represents a third emission surface of the third light control part 33, and "33o" is the center of the third emission surface 33b in top view. Also, "33x" represents a third axis, which passes through the center 33o of the third emission surface 33b and which is perpendicular to the third incident surface 33a. "33y" is the optical axis of the third light control parts 33. The optical axis 33y serves as a path where the light emitted from the center 23o of the light emitting surface 23a of the third light emitting part 23 travels. Note that FIG. 14 and FIG. 15 are cross sections that pass through the center 23o of the light emitting surface 23a of the third light emitting part 23 and include the third axis 33x.

As shown in FIG. 12 to FIG. 15, in each third stacked body S3, the third light control part 33 has a third incident surface 33a, which is planar in shape and which faces the third light emitting part 23, and a third emission surface 33b, which is curved to be convex in a direction away from the third incident surface 33a. In each third stacked body S3, the cross sections taken along the third axis 33x include a fourth cross section CS4, in which both sides of the third emission surface 33b with respect to the third axis 33x have curvatures different from each other.

In the example of the fourth cross section CS4 shown in FIG. 14, the part of the third emission surface 33b located nearer to the center region R1 shown in FIG. 3 relative to the third axis 33x (that is, the +Y half of the third emission surface 33b in FIG. 14) has a smaller curvature than that of the part of the third emission surface 33b located farther from the center region R1 shown in FIG. 3 relative to the third axis 33x (that is, the -Y half of the third emission surface 33b in FIG. 14).

When at least one third stacked body S3 thus includes a fourth cross section CS4, in which curvatures of both sides of the third emission surface 33b with respect to the third axis 33x are different from each other, the optical axis 33y of the third light control part 33 can be non-parallel to the third axis 33x. As a result of this, the light source module 1A can easily irradiate light onto a specific region.

A fourth cross section CS4 in which both sides of the third emission surface 33b with respect to the third axis 33x have curvatures different from each other may or may not be included in all of the third stacked bodies S3. By adjusting, in top view, the orientation of the fourth cross section CS4 in which curvatures of both sides of the third emission surface 33b with respect to the third axis 32x are different from each other in each third stacked body S3, light can be easily emitted from all of the third stacked bodies S3 toward a specific region.

The center 23o of the third light emitting surface 23a of the light emitting part 23 may or may not be on the fourth cross section CS4 in top view in at least one of the third stacked bodies S3. The center 23o of the light emitting surface 23a of the third light emitting part 23 may or may not be on the fourth cross section CS4 in top view in each of all of the third stacked bodies S3.

The third axis 33x may or may not pass through the center 23o of the light emitting surface 23a of the third light emitting part 23 in at least one of the third stacked bodies S3. The third axis 33x may or may not pass through the center 23o of the light emitting surface 23a of the third light emitting part 23 in each of all of the third stacked bodies S3.

In each of some or all of the third stacked bodies S3, the third axis 33x may or may not pass through the center 23o of the light emitting surface 23a of the third light emitting part 23. In other words, the center 33o and the center 23o may or may not coincide with each other in top view. For example, the positional relationship between a third light emitting part 23 and the corresponding third light control parts 33 can be adjusted within the range in which the center 23o of the light emitting surface 23a and the third emission surface 33b of the third light control parts 33 overlap with each other in top view. Adjusting the positional relationship between the center 33o and the center 23o in top view in addition to having curvatures different between both sides of the third emission surface 33b with respect to the first axis 31x in the fourth cross section CS4 allows for changing the extent that the optical axis 33y of the third light control part 33 is non-parallel to the third axis 33x.

In the fifth cross section CS5 shown in FIG. 15, both sides of the third emission surface 33b with respect to the third axis 33x have the same curvature. When circles corresponding to outer edges of adjacent light control parts have overlapping parts when viewed from above, such a shape is encompassed in the shape in which both sides of the third emission surface 33b with respect to the third axis 33x have the same curvature. Thus, in each third stacked body S3, the cross sections taken along the third axis 33x may include a fifth cross section CS5, which has the same curvature at both sides of the third emission surface 33b with respect to the third axis 33x and is perpendicular to the fourth cross section CS4. In all of the cross sections that are parallel to the fifth cross section CS5, both sides of the third emission surface 33b with respect to an axis that passes through the fourth cross section CS4 and that is perpendicular to the third incident surface 33a, may have the same curvature. Also, in all of the cross sections parallel to the fourth cross section CS4, the curvature of the third emission surface 33b may or may not different between both sides of the third emission surface 33b with respect to an axis that passes through the fifth cross section CS5 and that is perpendicular to the third incident plane 33a.

In FIG. 12 and FIG. 13, the arrow of the fourth cross section CS4 points to the side of the third emission surface 33b having the smaller curvature. In the example of FIG. 12, all the third stacked bodies S3 include a fourth cross section CS4 and a fifth cross section CS5. Further, in all of the third stacked bodies S3, the part of the third emission surface 33b located closer to the center region R1 shown in FIG. 3 relative to the third axis 33x has a smaller curvature than that of the third emission surface 33b located farther from the center region R1 shown in FIG. 3 relative to the third axis 33x. With this structure, the light source module 1A can emit light from all of the third stacked bodies S3 towards the center of the center region R1 with ease.

In FIG. 12, the extending line of the fourth cross section CS4 passes through the center region R1 shown in FIG. 3 in a top view in at least one of the third stacked bodies S3. The extending line of the fourth cross section CS4 may or may not pass through the center region R1 shown in FIG. 3 in top view in all of the third stacked bodies S3. In top view, the respective extending lines of the fourth cross sections CS4 of all third stacked bodies S3 may or may not intersect at one point. When the respective extending lines from the fourth cross sections CS4 of all third stacked bodies S3 intersect at one point, the lights emitted from individual third stacked bodies S3 can be concentrated in a narrower area.

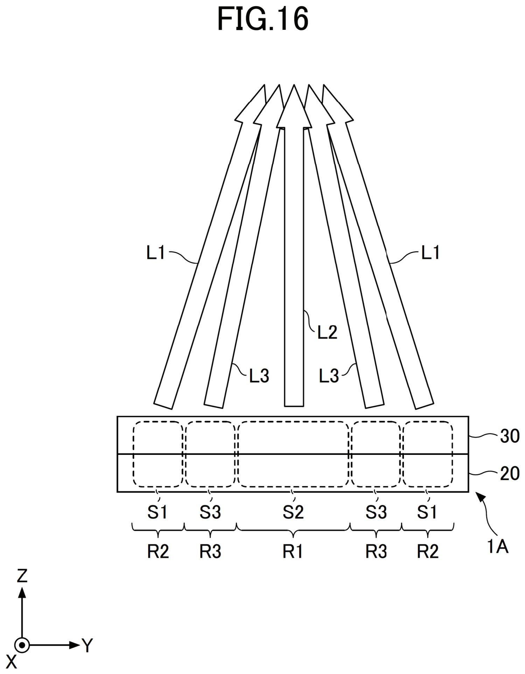

Thus, as shown schematically in FIG. 16, the configuration shown in FIG. 14 can facilitate emission of light from the first stacked bodies S1 toward the center of the center region R1, emission of light from the second stacked body S2 toward the center of the center region R1, and emission of light from the third stacked bodies S2 toward the center of the center region R1. In other words, the light source module 1A can concentrate a greater amount of light toward the center of the center region R1 than the light source module 1.

### <Second Modified Example of First Embodiment>

In a second modified example of the first embodiment, a light source module will be described below, in which the orientation of the first cross section CS1 in the first stacked bodies S1 is different from that of the first embodiment. In the following second modified example of the first embodiment, the second stacked body S2 and the third stacked bodies S3 are the same as those of the first embodiment.

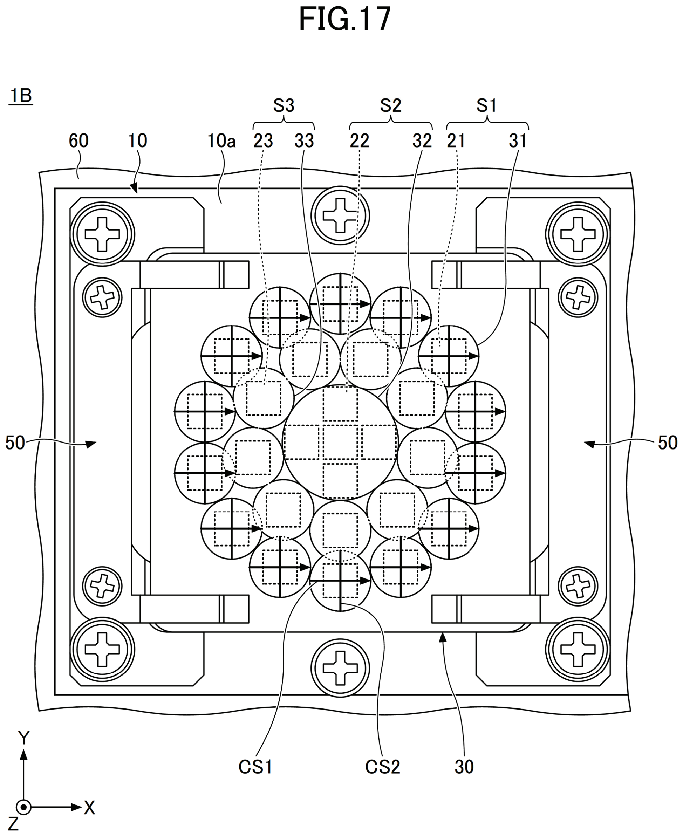

FIG. 17 is a partial top view showing an example of the light source module according to the second modified example of the first embodiment. In the light source module 1B, the optical axis of the first light control part 31 is non-parallel to the first axis 31x in all of the first stacked bodies S1. In the light source module 1B, all of the first stacked bodies S1 include a first cross section CS1 and a second cross section CS2. In a top view, the respective first cross sections CS1 of all first stacked bodies S1 are parallel to each other or coplanar with each other. The positional relationship between the part of the first emission surface 31b having the larger curvature and the part having the smaller curvature with respect to the first axis 31x is the same among all of the first cross sections CS1.

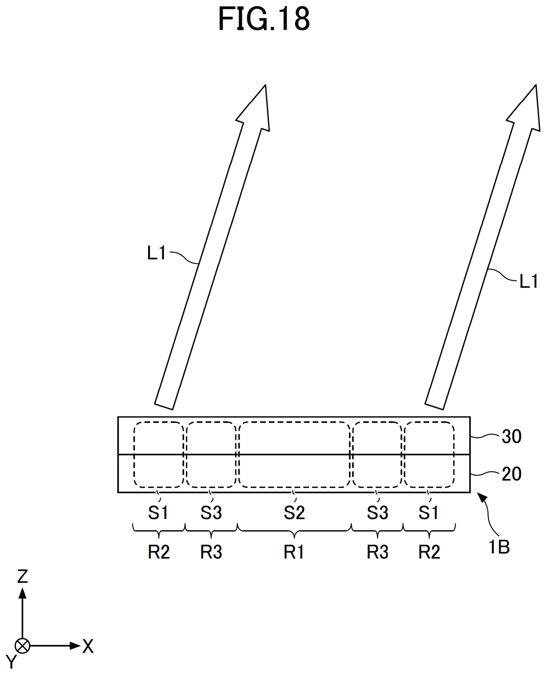

In the example of FIG. 17, the part of the first emission surface 31b located in the +X direction relative to the first axis 31x has a smaller curvature than that of the part of the first emission surface 31b located in the -X direction relative to the first axis 31x. With this structure, in the light source module 1B, light can be easily irradiated from all of the first stacked bodies S1 in a specific direction. To be more specific, as schematically illustrated in FIG. 18, each first stacked body S1 can emit light diagonally upward in the +X direction with ease. Each first stacked body S1 has an optical axis that is parallel to the first cross section CS1 in a top view, and the lights emitted from individual first stacked bodies S1 travel from the -X side to the +X side in a top view. In the light source module 1B, making the first cross section CS1 parallel to a specific direction in top view allows each first stacked body S1 to emit light that is parallel to the first cross section CS1 in a top view, in a specific direction.

### <Third Modified Example of First Embodiment>

In a third modified example of the first embodiment, a light source module will be described below, in which the orientation of the fourth cross section CS4 in the third stacked bodies S3 is different from that of the second modified example of the first embodiment. In the third modified example of the first embodiment, the second stacked body S2 and the third stacked bodies S3 are the same as those of the first embodiment.

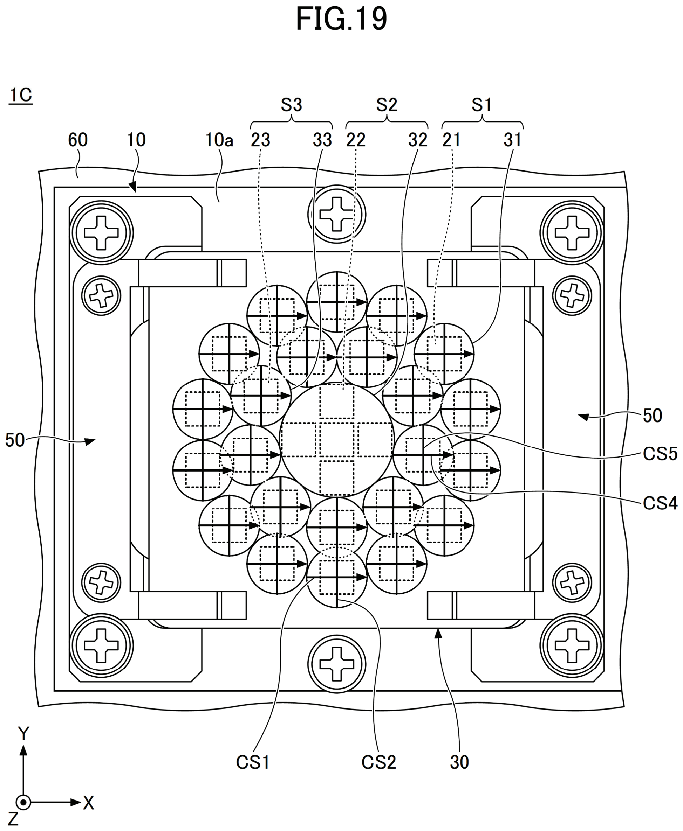

FIG. 19 is a partial top view showing an example of the light source module according to the third modified example of the first embodiment. In the light source module 1C, the optical axis of the third light control part 33 is non-parallel to the third axis 33x in all of the third stacked bodies S3. In the light source module 1C, all of the third stacked bodies S3 include a fourth cross section CS4 and a fifth cross section CS5. In top view, the respective fourth cross sections CS4 of all third stacked bodies S3 are parallel to the first cross section CS1 or on the same plane. In all of the fourth cross sections CS4, the positional relationship between the part of the third emission surface 33b having the larger curvature relative to the third axis 33x and the part having the smaller curvature is the same as the positional relationship between the part of the first emission surface 31b having the larger curvature relative to the first cross section CS1 and the part having the smaller curvature in the first cross section CS1.

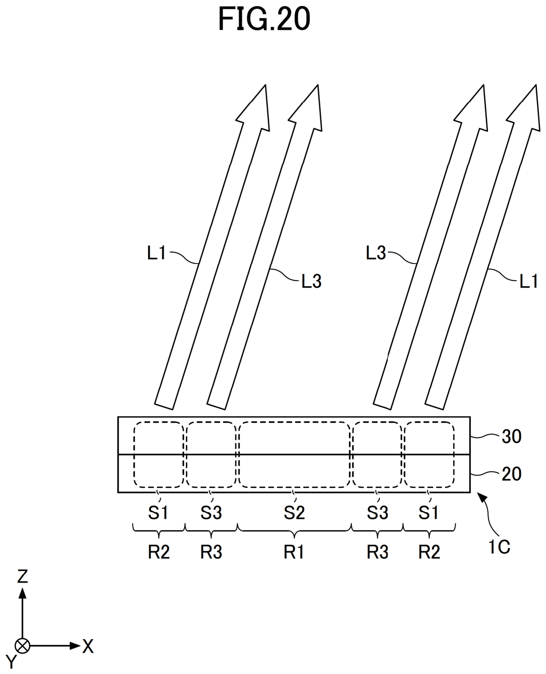

In the example of FIG. 19, the part of the third emission surface 33b located in the +X direction relative to the third axis 33x has a smaller curvature than that of the part of the third emission surface 33b located in the -X direction relative to the third axis 33x. With this structure, the light source module 1B allows all of the first stacked bodies S1 to emit light in a specific direction with ease. To be more specific, as schematically illustrated in FIG. 20, each first stacked body S1 and third stacked body S3 can emit light diagonally upward in the +X direction with ease. Each first stacked body S1 and each third stacked body S3 have an optical axis that is parallel to the first cross section CS1 and the fourth cross section CS4 in a top view, and the lights emitted from individual first stacked bodies S1 and individual third stacked bodies S3 travel from the - X side to the +X side in a top view. In the light source module 1C, with the first cross section CS1 and the fourth cross section CS4 parallel to a specific direction in top view, it is possible to allow each first stacked body S1 and each third stacked body S3 to emit light that is parallel to the first cross section CS1 and the fourth cross section CS4 in a top view, in a specific direction. In other words, the light source module 1C can emit a greater amount of light in a specific direction than the light source module 1B.

### <Fourth Modified Example of First Embodiment>

In a fourth modified example of the first embodiment, an example in which separate light control parts are used will be described below. In the description given thus far, examples have been shown in which an optical member having multiple light control parts is used in a light source module. However, a light source module may use multiple optical members that each have one light control part.

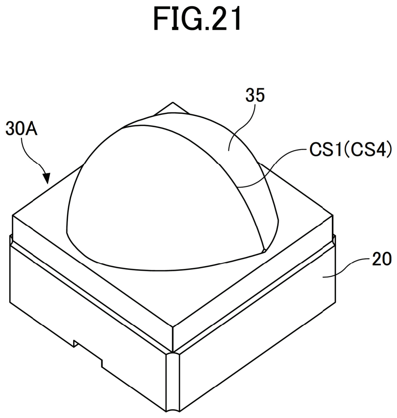

FIG. 21 is a perspective view showing an example of an optical member for use in the light source module according to the fourth modified example of the first embodiment. As shown in FIG. 21, an optical member 30A include one light control part 35 positioned above one light emitting part in the light sources 20. The light control part 35 may include, for example, a first cross section CS1 or a fourth cross section CS4. In the example of FIG. 21, CS1 (CS4) indicates the position of a first cross section CS1 or a fourth cross section CS4. The line of CS1 (CS4) is a virtual line.

The light sources 20 may be, for example, a light emitting device in which one light emitting element is provided in a package. In this case, the first stacked bodies S1 and third stacked bodies S3 can be formed by using optical members 30A. The light sources 20 may also be a light emitting device in which one or more light emitting elements are provided in a package. In this case, a second stacked body S2 can be formed by using an optical member 30A. By positioning these optical members 30A, for example, as shown in FIG. 2, a light source module can be formed.

### <Second Embodiment>

In a second embodiment of the present disclosure, an example of an ultraviolet light fluid processing device having the light source module of the first embodiment will be shown.

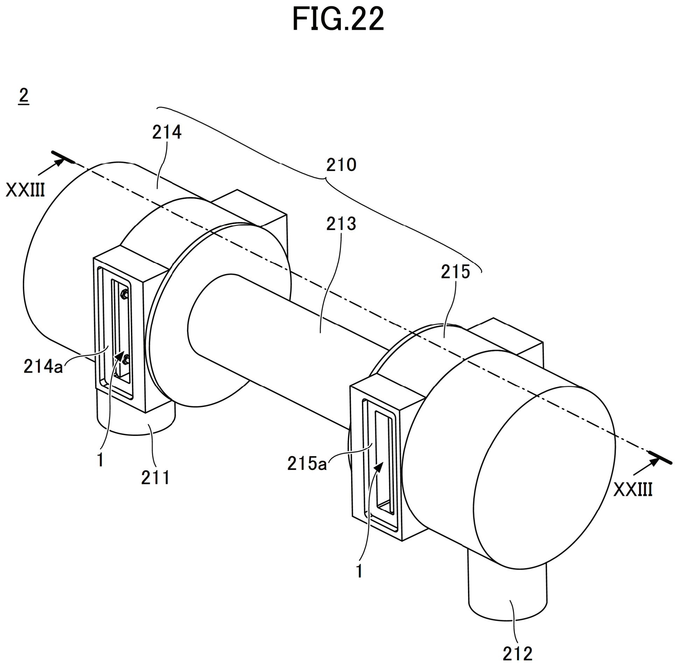

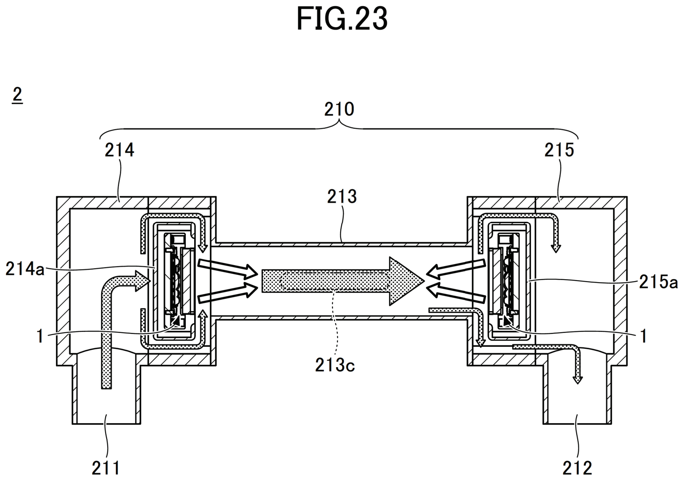

FIG. 22 is a perspective view showing an example of the ultraviolet light fluid processing device according to the second embodiment. FIG. 23 is a cross-sectional view taken along the line XXIII-XXIII in FIG. 22. Note that, in FIG. 23, the dotted arrows are schematic representations of the flow of fluid. Also, the blank arrows (arrows without dots) are schematic representations of the directions in which the light source module emits ultraviolet light.

As shown in FIG. 22 and FIG. 23, the ultraviolet light fluid processing device 2 has a flow pipe 210, in which fluid flows, and two light source modules 1 that can irradiate the ultraviolet light exiting the optical member 30 (that is, emit ultraviolet light to) onto the inside of the flow pipe 210. The ultraviolet light fluid processing device 2 may include one or three or more light source modules 1.

The flow pipe 210 includes: a flow-in part 211 and a flow-out part 212 where fluid enters and exits the flow pipe 210, respectively; a flow part 213 in which the fluid flows; a first connecting part 214 connecting the flow-in part 211 and the flow part 213; and a second connecting part 215 connecting the flow part 213 and the flow-out part 212. Fluid enters the flow pipe 210 from the flow-in part 211, travels across the flow part 213, and exits from the flow-out part 212. The direction in which the fluid flows may be reversed.

The first connecting part 214 is provided with a light source positioning part 214a for inserting one of the light source modules 1. The light source positioning part 214a is positioned such that the direction in which the one of the light source modules 1 is inserted in the light source positioning part 214a is substantially perpendicular to the direction in which the fluid enters the flow pipe 210 from the flow-in part 211, and the optical members 30 of the inserted light source module 1 face the flow part 213. The one light source module 1 is thus inserted in the light source positioning part 214a and fixed in the light source positioning part 214a by the spring members 70 (see FIG. 1).

The second connecting part 215 is provided with a light source positioning part 215a for inserting the other light source module 1. The light source positioning part 215a is positioned such that the direction in which the other light source module 1 is inserted in the light source positioning part 215a is substantially perpendicular to the direction in which the fluid exits the flow pipe 210 from the flow-out part 212, and the optical members 30 of the inserted light source module 1 face the flow part 213. The other light source module 1 is inserted in the light source positioning part 215a and fixed in the light source positioning part 215a by the spring members 70 (see FIG. 1).

The lower surface of the holding member 60 being a part of one light source module 1 can be positioned in surface contact with an inner wall of the light source positioning part 214a (see FIG. 1). Likewise, the lower surface of the holding member 60 being a part of the other light source module 1 can be positioned in surface contact with an inner wall of the light source positioning part 215a (see FIG. 1). With this structure, the heat produced while the light source modules are in operation can be dissipated to the fluid flowing around the light source positioning parts.

The flow part 213 extends in the direction normal to the upper surface 10a of the substrate 10 of one light source module 1 (see FIG. 1). Also, the flow part 213 extends in the direction normal to the upper surface 10a of the substrate 10 of the other light source module 1 (see FIG. 1). Each light source module 1 can irradiate the ultraviolet light exiting the optical members 30 onto the inside of the flow part 213.

Thus, in the examples shown in FIG. 22 and FIG. 23, the ultraviolet light fluid processing device 2 can emit ultraviolet light, from the two light source modules 1, to the fluid in the flow part 213. With this structure, the ultraviolet light fluid processing device 2 can process the fluid by irradiating the fluid in the flow part 213 with ultraviolet light, thereby, for example, reducing the number of bacteria and viruses in the processed water compared to before the processing. Note that "fluid" used herein may include liquids, gases, etc.

The flow-in part 211 and the flow part 213 are located opposite each other with respect to the light source positioning part 214a. Between the outer walls of the light source positioning part 214a and the inner walls of the first connecting part 214, an upper gap and a lower gap that are opposite each other with respect to the light source positioning part 214a are formed. The fluid enters the first connecting part 214 from the flow-in part 211, travels in the upper and lower gaps formed opposite each other with respect to the light source positioning part 214a and enters the flow part 213.

Also, the flow-out part 212 and the flow part 213 are located opposite each other with respect to the light source positioning part 215a. Between the outer walls of the light source positioning part 215a and the inner walls of the second connecting part 215, an upper gap and a lower gap that are opposite each other with respect to the light source positioning part 215a are formed. The fluid enters the second connecting part 215 from the flow part 213, travels in the upper and lower gaps formed opposite each other with respect to the light source positioning part 215a and enters the flow-out part 212.

Looking at the flow rate (i.e., speed) of the fluid in the flow part 213, the flow rate in the center part in the flow part 213 may be faster than the flow rate in the outer parts, or the flow rate in the outer parts in the flow part 213 may be faster than the flow rate in the center part. Referring to the example of FIG. 23, the branch of the fluid that flows above the upper outer wall of the light source positioning part 214a and then enters the flow part 213 and the branch of the fluid that flows below the lower outer wall of the light source positioning part 214a and then enters the flow part 213 merge in the center part 213c in the flow part 213, so that the flow rate in the center part 213c is faster than the flow rate in the outer parts in the flow part 213. By using the light source modules 1 that can collect light in the center part 213c in the flow part 213 thus, an increased amount of ultraviolet light can be collected in the center part 213c in the flow part 210, thereby improving the sterilization performance of the ultraviolet light fluid processing device 2. Note that light source modules 1A may be used instead of the light source modules 1. By using light source modules 1A, a further increased amount of ultraviolet light can be collected in the center part 213c in the flow part 213, thereby further improving the sterilization performance of the ultraviolet light fluid processing device 2.

Also, by using the light source modules 1, light is collected in the center part 213c in the flow part 213, so that vignetting caused by the light source positioning parts can be reduced.

### <First Modified Example of Second Embodiment>

In a first modified example of the second embodiment, an ultraviolet light fluid processing device with different light source modules from those of the second embodiment described above will be described. In the ultraviolet light fluid processing device of the first modified example of the second embodiment, the direction in which the light source modules are inserted, relative to the direction in which fluid flows in and out of the flow part 210, is different from that in the ultraviolet light fluid processing device of the second embodiment described above.

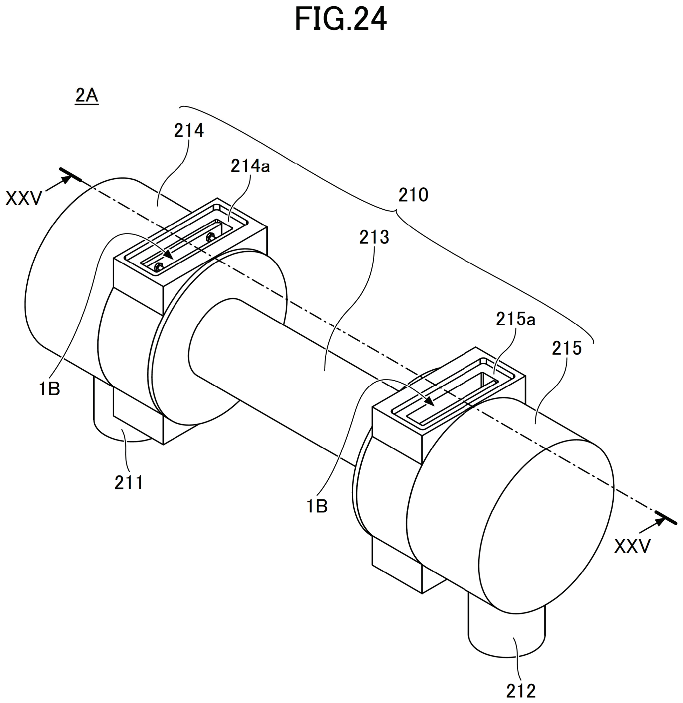

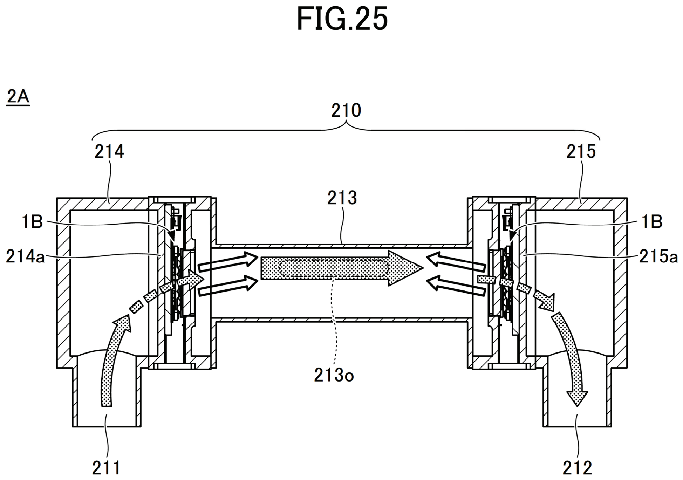

FIG. 24 is a perspective view showing an example of the ultraviolet light fluid processing device according to the first modified example of the second embodiment. FIG. 25 is a cross-sectional view taken along the line XXV-XXV in FIG. 24. In FIG. 25, the dotted arrows are schematic representations of the flow of fluid. Also, the blank arrows (arrows without dots) are schematic representations of the directions in which the light source modules emit ultraviolet light. Referring to FIG. 24 and FIG. 25, the ultraviolet light fluid processing device 2A is different from the ultraviolet light fluid processing device 2 in that the two light source modules 1 of the ultraviolet light fluid processing device 2 are replaced with two light source modules 1B.

Also, unlike the ultraviolet light fluid processing device 2, the light source positioning part 214a of the ultraviolet light fluid processing device 2A is positioned such that the direction in which one light source module 1B is inserted in the light source positioning part 214a is substantially parallel to the direction in which fluid enters the flow pipe 210 from the flow-in part 211, and the optical members 30 of the inserted light source module 1B are oriented to face the flow part 213. Also, the light source positioning part 215a is positioned such that the direction in which the other light source module 1B is inserted in the light source positioning part 215a is substantially parallel to the direction in which the fluid exits the flow pipe 210 from the flow-out part 212, and the optical members 30 of the inserted light source module 1B are oriented to face the flow part 213.

In the example of FIG. 25, the flow-in part 211 and the flow part 213 are located opposite each other with respect to the light source positioning part 214a. Between the outer walls of the light source positioning part 214a and the inner walls of the first connecting part 214, a front gap and a rear gap that are opposite each other are formed in front of and behind the light source positioning part 214a. The fluid enters the first connecting part 214 from the flow-in part 211, travels in the front and rear gaps formed opposite each other in front of and behind the light source positioning part 214a and enters the flow part 213.

Also, the flow-out part 212 and the flow part 213 are located opposite each other with respect to the light source positioning part 215a. Between the outer walls of the light source positioning part 215a and the inner walls of the second connecting part 215, a front gap and a rear gap that are opposite each other are formed in front of and behind the light source positioning part 215a. The fluid enters the second connecting part 215 from the flow part 213, travels in the front and rear gaps formed opposite each other with respect to the light source positioning part 215a and enters the flow-out part 212.

The fluid enters the first connecting part 214 from the flow-in part 211, travels in front of and behind the outer walls of the light source positioning part 214a and enters the flow part 213. Referring to FIG. 25, the arrows' dashed parts represent the branches of fluid that travel in front of and behind the outer walls of the light source positioning part 214a. Referring to FIG. 25, the branch of the fluid that flows in front of the outer walls of the light source positioning part 214a and then enters the flow part 213 and the branch of the fluid that flows behind the outer walls of the light source positioning part 214a and then enters the flow part 213 merge in the outer-circumferential upper part 213o in the flow part 213, so that the flow rate in the outer-circumferential upper part 213o is faster than the flow rate in the center part and the outer-circumferential lower part in the flow part 213. The above-mentioned merging occurs in the outer-circumferential upper part 213o in the flow part 213 because no branch of fluid travels above the upper outer wall of the light source positioning part 214a and then enters the flow part 213 as is the case of the example of FIG. 22. By using the light source modules 1B that can concentrate light in the outer-circumferential upper part 213o in the flow part 213 thus, an increased amount of ultraviolet light can be collected in the outer-circumferential upper part 213 in the flow part 210, thereby allowing for improvement in the sterilization performance of the ultraviolet light fluid processing device 2A. Light source modules 1C may be used instead of the light source modules 1B. By using light source modules 1C, a further increased amount of ultraviolet light can be collected in the outer-circumferential upper part 213 in the flow part 210, thereby further improving the sterilization performance of the ultraviolet light fluid processing device 2A.

Preferred embodiments of the present disclosure, as well as their modified examples, have been described in detail above. However, the present disclosure is by no means limited to the above-described embodiments and modified examples, and a variety of modifications, substitutions, etc. can be made to the embodiments and modified examples without departing from the technical scope defined by the accompanying claims.

For example, the light control parts described herein may be cylindrical lenses or Fresnel lenses.

## Claims

1. A light source module comprising:
a substrate having a center region and an outer region located outward of the center region;
light sources positioned on an upper surface of the substrate and comprising a plurality of first light emitting parts; and
an optical member positioned above the substrate such that the light sources are located between the substrate and the optical member, the optical member comprising light control parts that comprise a plurality of first light control parts,
wherein each of the plurality of first light control parts overlaps with a respective one of the plurality of first light emitting parts in a top view so as to constitute each of a plurality of first stacked bodies positioned in the outer region,
wherein, in each of the plurality of first stacked bodies, the first light control part comprises:
a first incident surface that is planar in shape and that faces a corresponding first light emitting part of the plurality of first light emitting parts; and
a first emission surface that is curved to be convex in a direction away from the first incident surface,
wherein, in each of the plurality of first stacked bodies, cross sections along a first axis that passes through a center of the first emission surface in the top view and that is perpendicular to the first incident surface include a first cross section in which curvatures of both sides of the first emission surface with respect to the first axis are different from each other, and
wherein, in at least one of the plurality of first stacked bodies, an optical axis of the corresponding first light control part is non-parallel to the first axis.

2. The light source module according to claim 1, wherein, in at least one of the plurality of first stacked bodies, in the top view, a center of a light emitting surface of the corresponding first light emitting part is included in the first cross section.

3. The light source module according to claim 1,
wherein, in each one of the plurality of first stacked bodies, the cross sections along the first axis include a second cross section in which both sides of the first emission surface with respect to the first axis have a same curvature, the second cross section being perpendicular to the first cross section, and
wherein, in at least one of the plurality of first stacked bodies, in the top view:
an extending line of the first cross section passes through the center region of the substrate; and
in the first cross section, a side of the first emission surface located closer to the center region relative to the first axis has a smaller curvature than a side of the first emission surface located farther from the center region relative to the first axis.

4. The light source module according to claim 3,
wherein, in each of the plurality of first stacked bodies, an optical axis of the first light control part is non-parallel to the first axis,
wherein, in each of the plurality of first stacked bodies, the cross sections including the first axis include the second cross section, and
wherein, in each of the plurality of first stacked bodies, in top view:
the extending line of the first cross section passes through the center region of the substrate; and
in the first cross section, a side of the first emission surface located closer to the center region relative to the first axis has a smaller curvature than a side of the first emission surface located farther from the center region relative to the first axis.

5. The light source module according to claim 1,
wherein, in each of the plurality of first stacked bodies, an optical axis of the first light control part is non-parallel to the first axis,
wherein, in each of the plurality of first stacked bodies, the cross sections including the first axis include a second cross section in which both sides of the first emission surface with respect to the first axis have a same curvature, the second cross section being perpendicular to the first cross section,
wherein, a plurality of said first cross sections, each included in a respective one of the plurality of first stacked bodies, are parallel to each other in top view, and
wherein, a positional relationship between a side of the first emission surface having a larger curvature and a side of the first emission surface having a smaller curvature with respect to the first axis is the same across the plurality of said first cross sections.

6. The light source module according to claim 1,
wherein the light sources further include one or more second light emitting parts,
wherein the light control parts further include one or more second light control parts,
wherein the one or more second light control parts overlap with the one or more second light emitting parts in the top view so as to constitute one or more second stacked bodies, a number of the one or more second stacked bodies being the same as the number of the one or more second light control parts,
wherein the second stacked bodies are positioned in the center region, and
wherein, in each of the second stacked bodies, a corresponding second light control part among the one or more second light control parts includes:
a second incident surface that is planar in shape and that faces a corresponding second light emitting part of the one or more second light emitting parts; and
a second emission surface that is curved to be convex in a direction away from the second incident surface.

7. The light source module according to claim 6,
wherein the light sources further include a plurality of third light emitting parts,
wherein the light control parts further include a plurality of third light control parts,
wherein each of the plurality of third light control parts overlaps with a respective one of the plurality of third light emitting parts in the top view so as to constitute each of a plurality of third stacked bodies,
wherein the substrate has a middle region between the center region and the outer region,
wherein the plurality of third stacked bodies are positioned in the middle region,
wherein, in each of the plurality of third stacked bodies, the third light control part comprises:
a third incident surface that is planar in shape and that faces a corresponding third light emitting part of the plurality of third light emitting parts; and
a third emission surface that is curved to be convex in a direction away from the third incident surface,
wherein, in each of the plurality of third stacked bodies, cross sections including a third axis that passes through a center of the third emission surface in the top view and that is perpendicular to the third incident surface include a fourth cross section in which curvatures of both sides of the third emission surface with respect to the third axis are different from each other, and
wherein, in at least one of the plurality of third stacked bodies, an optical axis of the third light control part is non-parallel to the third axis.

8. The light source module according to claim 7, wherein, in at least one of the plurality of third stacked bodies, a center of a light emitting surface of the third light emitting part is included in the fourth cross section in top view.

9. The light source module according to claim 8,
wherein, in each of the plurality of third stacked bodies, the cross sections including the third axis include a fifth cross section in which both sides of the third emission surface with respect to the third axis have a same curvature, the fifth cross section being perpendicular to the fourth cross section, and
wherein, in the at least one of the plurality of third stacked bodies, in top view:
an extending line of the fourth cross section passes through the center region of the substrate; and
in the fourth cross section, a side of the third emission surface located nearer to the center region relative to the third axis has a smaller curvature than a side of the third emission surface located farther from the center region relative to the third axis.

10. The light source module of claim 9,
wherein, in each of the plurality of third stacked bodies, an optical axis of the third light control part is non-parallel to the third axis,
wherein, in each of the plurality of third stacked bodies, the cross sections each taken along the third axis include the fifth cross section, and
wherein, in each of the plurality of third stacked bodies, in the top view:
the extending line of the fourth cross section passes through the center region of the substrate; and
in the fourth cross section, the side of the third emission surface located closer to the center region relative to the third axis has a smaller curvature than the side of the third emission surface located farther from the center region relative to the third axis.

11. The light source module according to claim 10,
wherein the light sources further include one or more second light emitting parts,
wherein the light control parts further include one or more second light control parts,
wherein the one or more second light control parts and the one or more second light emitting parts overlap with each other in top view so as to constitute one or more second stacked bodies a number of the second stacked bodies being the same as the number of the one or more second light control parts,
wherein the second stacked bodies are positioned in the center region,
wherein, in each of the second stacked bodies, the second light control part comprises:
a second incident surface that is planar in shape and that faced a corresponding second light emitting part among the one or more second light emitting parts; and
a second emission surface that is curved to be convex in a direction away from the second incident surface.

12. The light source module according to claim 11,
wherein the light sources further include a plurality of third light emitting parts,
wherein the light control parts further include a plurality of third light control parts,
wherein each of the plurality of third light control parts overlap with a respective one of the plurality of third light emitting parts in the top view so as to constitute a respective one of a plurality of third stacked bodies,
wherein the substrate has a middle region between the center region and the outer region,
wherein the plurality of third stacked bodies are positioned in the middle region,
wherein, in each of the plurality of third stacked bodies, the third light control part comprises:
a third incident surface that is planar in shape and oriented to face a corresponding third light emitting part among the plurality of third light emitting parts; and
a third emission surface that is curved to be convex in a direction away from the third incident surface,
wherein, in each of the plurality of third stacked bodies, cross sections each taken along a third axis that passes through a center of the third emission surface in top view and that is perpendicular to the third incident surface include a fourth cross section in which curvatures of both sides of the third emission surface with respect to the third axis are different from each other, and
wherein, in at least one of the plurality of third stacked bodies, an optical axis of the third light control part is non-parallel to the third axis.

13. The light source module of claim 12,
wherein, in each of the plurality of third stacked bodies, an optical axis of the corresponding third light control part is non-parallel to the third axis,
wherein, in each of the plurality of third stacked bodies, the cross sections including the third axis include a fifth cross section in which both sides of the third emission surface with respect to the third axis have a same curvature, the fifth cross section being perpendicular to the fourth cross section,
wherein, a plurality of said fourth cross sections, each included in a respective one of the plurality of third stacked bodies, are parallel to the first cross section in top view, and
wherein the positional relationship between a side of the third emission surface having a larger curvature and a side of the third emission surface having a smaller curvature, with respect to the third axis, is the same across the plurality of the fourth cross sections.

14. The light source module according to one of claim 1 to claim 13, wherein ultraviolet light exits the optical member.

15. An ultraviolet light fluid processing device comprising:
a flow pipe in which a fluid flows; and
the light source module of claim 14, configured to irradiate the ultraviolet light exited from the optical member onto inside of the flow pipe.
